# EUROPEAN PATENT APPLICATION

(11) **EP 4 621 642 A1**
(43) Date of publication of application: **24.09.2025**
(21) Application number: 24165477.1
(22) Date of filing: 22.03.2024
(51) Int. Cl.: G06M 1/00, G06M 1/14, G06M 1/16, G06M 1/24, G06M 1/26, A61M 15/00

(54) **COUNTING ARRANGEMENT FOR A MEDICAL DISPENSER DEVICE**

(71) Applicant: Sandoz International Gmbh, 83607 Holzkirchen (DE)
(72) Inventor: CHRISTIE, Ewen, Cambridge (GB); COCKER, Robin, Cambridge (GB)
(74) Representative: Maiwald GmbH

(57) **Abstract**

The present invention relates to a counting arrangement for counting binary mechanical inputs comprising: a rotatably mounted units wheel (10) arranged to rotate by one unit for every input that is to be counted; a tens wheel (20) in mechanical communication with the units wheel (10), and in part overlapping the units wheel (10) in order to provide an at least two-digit display; a rotation control mechanism, comprising: at least one circular rail (11; 12) on the units wheel (10) with at least two substantially radial rail gaps (13, 14); a pins wreath (21) arranged on the tens wheel (20) consisting of several axial pins (21a - 21e) equidistantly arranged in a circle to one another for engaging with the circular rail (11; 12); a units wheel indexing cam face (15) that engages with the pins wreath (21) in order to effect driving of the tens wheel (20) by one increment during every tenth increment of the units wheel (10); wherein the at least one circular rail (11, 12) on the units wheel (10) locks the tens wheel (20) in a configuration in which it cannot rotate during the intermediate nine inputs; wherein the position of the tens wheel axis (22) is defined in the radial direction (R) by a first, radially-constraining component, and defined in the tangential direction (T) by a separate second, tangentially-constraining component.

## Description

The present invention relates to an improved counting arrangement for use in a medicament dispenser device.

Many medicament dispenser devices require a means to display the number of doses of medicament that remain within the device or the number of doses that have been dispensed. This count allows a user, or a medical professional, to track usage of the device within the unit itself, to ensure that the device is being used as prescribed, and to alert as to how many doses remain to ensure that the user always has a medicament available. These medicament dispensers include inhalers, such as dry powder inhalers, metered dose inhalers, and similar devices.

### Prior art

A number of these already known medicament dispensers incorporate a mechanical or electronic counting device to keep count of a number of doses that remain or that have been dispensed. Typically, these medicament dispensers are required to be available to a user at all times, and therefore they are often transported in a pocket, handbag or otherwise. The dispenser, and therefore the counting device contained therein, needs to be relatively robust to cope in these environments when subjected to adverse conditions, which might include various impacts and vibrations, and the attempted ingress of dirt and other detritus. However, there is also a trade-off that whilst being robust they need to be relatively compact and space-efficient, making them easy to transport so that a user is encouraged to carry them on their person or in with their essential items at all times. Likewise, the counting device should not take up excessive space within the dispenser itself.

It is vital that where a counting device is included within the dispenser, the count displayed on the device at any one time is accurate. An inaccurate count displayed on the device can lead to confusion as to whether a prescribed dosage has been followed, wastage of medicament when a device is disposed of before a medicament has run out, or a potentially dangerous situation where the medicament has run out although the count reads that there should be some available, and a user is therefore without the dose that they need. Whilst electronic counting devices typically overcome this problem of unwanted movement of mechanical parts leading to an inaccurate count, they add considerable cost to what is often a disposable item. However, mechanical counting devices, whilst typically providing a more cost-effective option that is also not reliant on a power source, can often be affected by any unwanted movement.

The number of component parts within a dose counter can also have an effect on the likelihood of failure, or of the count being inaccurate. Typically, lots of component parts can also mean a more expensive device to manufacture and assemble. The market for medicament dispensers is greatly affected by cost, with manufacturers seeking ways to reduce unit cost wherever possible.

Another problem with existing devices is the size of the count that is displayed to the user of the counting device. The count is often viewed by a user through a small window in a housing of the counting device, and the size of the number that is displayed can be hard to read, particularly for those mechanical counting devices that incorporate a single wheeled device that displays a count value. To allow the wheel to be of a suitable size to fit within the body of a typical inhaler, it must be of a suitable diameter. This can leave little room in which to fit numbers that count up to or down from sixty, one hundred, or a value even more than that for some medicament dispensers. Those counting devices that comprise a units wheel and a tens wheel attempt to deal with this issue. However, typically these require side-by-side wheels, or a concentric arrangement of wheels, and again to fit this within the body of a typical medicament dispenser, the wheels must be as small as possible, leaving little space in which to fit the numbers.

In order to present a coherent and comprehensible display to the user, it is important that the appearance of unwanted digits - for example, the digits either side of the intended digit on the display wheels - in the count window of the device is avoided or minimized, while desired digits also remain within the window at all times. Higher tolerances in the mechanism and in the printing (or other form of rendering) of digits on the wheels will result in higher tolerances on the digit position, meaning that the chance of unwanted digits appearing in the count window, or the desired digits moving partially out of the window, is increased. Higher tolerances will therefore necessitate a smaller window to avoid the appearance of unwanted digits, and smaller digits to avoid the desired digits moving out of the window. The smaller window will further compound the requirement for smaller digits, as a smaller deviation would thus be required for the edge of the digits to move out of the window. Higher tolerances thus have a disproportionate effect on the possible size of the digits, and thus on the legibility and clarity of the count.

WO 2016/092252 A1 discloses a mechanical counting arrangement comprising a rotatably mounted first counting wheel arranged to rotate by a predetermined amount for every input that is to be counted; a second counting wheel in mechanical communication with the first counting wheel, and at least in part overlapping the first counting wheel, wherein the second counting wheel and/or the first counting wheel are at least in part transparent; whereby the mechanical communication between the first counting wheel and the second counting wheel comprises a rotation control mechanism configured to drive the second counting wheel a predetermined amount when a predetermined number of inputs has been reached.

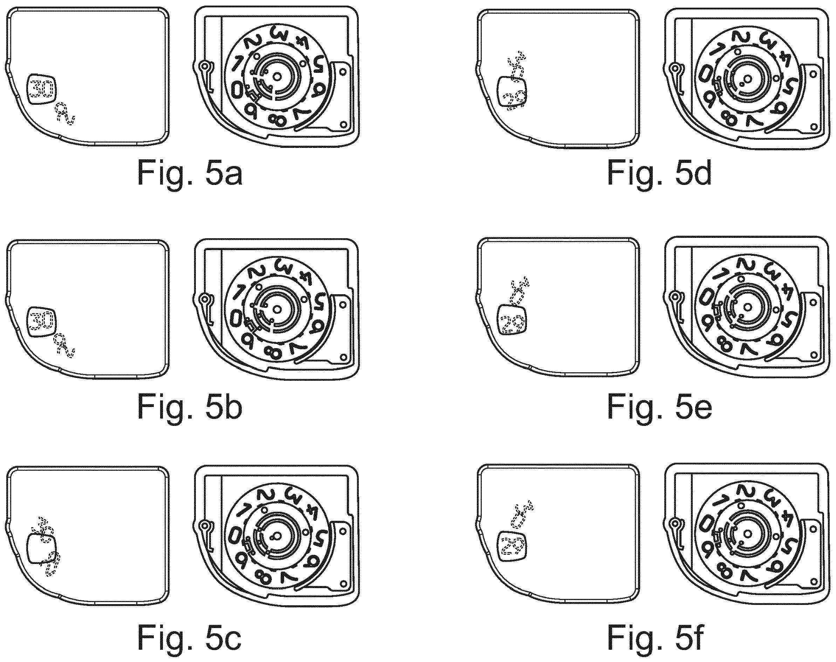

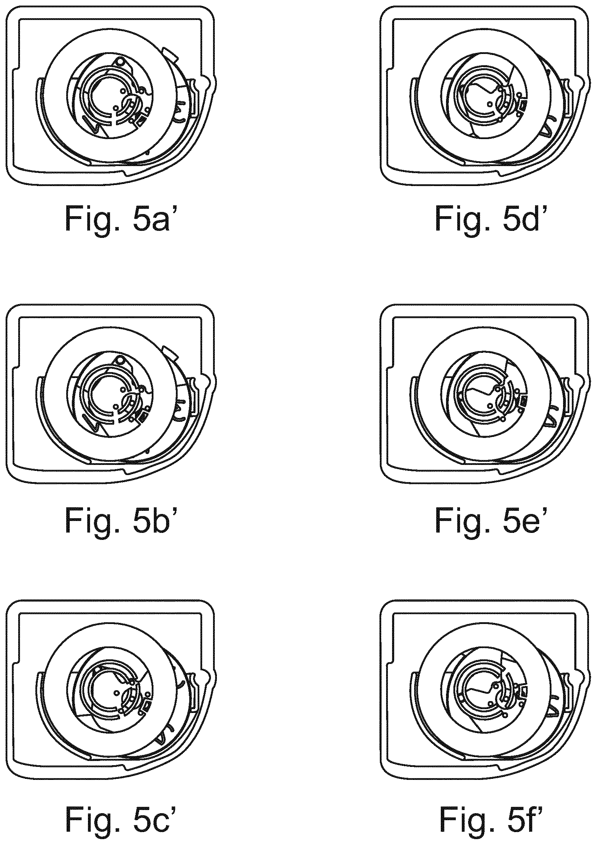

According to a pin rail version (Fig. 5 ff.) of several embodiments of counting arrangements disclosed in the said prior art document, the rotation control mechanism comprises a circular rail on the units wheel with two substantially radial gaps which communicate with a pins wreath arranged on the tens wheel consisting of several axial pins equidistantly arranged in a circle to one another for engaging with the circular rail on the units wheel. The units wheel further comprises an indexing cam face that engages with the pins wreath in order to effect driving of the tens wheel by one increment during every tenth increment of the units wheel. The circular rail on the units wheel locks the tens wheel in a configuration in which it cannot rotate during the intermediate nine inputs.

The rotation control mechanism is activated by the pins wreath of the tens wheel contacting the units wheel indexing cam face. The tens wheel is free to rotate because of the activation of release mechanism through alignment of the units wheel radial rail gaps with some of the pins in the pins wreath, allowing the pins to pass through the rail. The tens wheel is free to rotate by one tens wheel gear increment such that a different tens wheel numeral is shown through housing window.

For a reliable function of the dose counter, it is necessary that the few single parts are manufactured with high precision, in particular to avoid jams during the tens wheel switching by another increment. High precision, that is to say low manufacturing tolerances, also allows digits that are both larger and better aligned, improving the legibility and clarity of the count.

It is an object of the present invention to provide a counting arrangement for counting binary mechanical inputs comprising at least a units wheel and a tens wheel which allows lower mechanism tolerances in order to avoid jamming and/or poor display quality.

### Summary of the invention

This object is solved by a counting arrangement according to claim 1. Claim 13 is directed to a medical dispenser device which is equipped with a counting arrangement according to the present invention.

According to the invention, the position of the tens wheel axis is substantially defined in the radial direction R by a first, radially-constraining component, and substantially defined in the tangential direction T by a separate second, tangentially-constraining component.

In other words, the present invention provides a pin rail counter arrangement in which the radial position of the tens wheel axis is defined by the units wheel, wherein the axial position of the tens wheel axis is defined by both the housing component and the units wheel. This specific guiding solution allows much shorter tolerance chains governing the interactions of the tens wheel pins and the units wheel rails. The radial position of the tens wheel is directly governed by the axis held in the circumferential gap between the units wheel rails. This means that the tolerance chain governing the interaction of the other tens wheel pins with the units wheel rails only contains dimensions from the two wheels, rather than the dimensions from other components that would otherwise dictate the radial position of the two wheels relative to each other. This in turn means that the tolerance-mandated gaps between the pins and rails can be considerably tighter, and the mechanism and the digit alignment is therefore more tightly controlled.

At any moments of mechanism operation in which the tens wheel axis is not held by the interaction of the rails of the units wheel with the axis of the tens wheel, it is sufficiently held by the interaction of the rails with the other pins.

Preferably, the said first, radially-constraining component is a circumferential units wheel tens-positioning cam. The said separate second, tangentially-constraining component is preferably provided by a slot in a housing component of the counting arrangement.

Because of the shorter tolerance chain between the axis positions of the units and tens wheel, and thus lower overall mechanism tolerances, a superior count display can be achieved with larger digits and better digit alignment. Alternatively, the same quality of count display can be achieved while using larger manufacturing tolerances for individual components, allowing different manufacturing processes to be used and/or cheaper component manufacturing. Alternatively, the same quality of count display can be achieved with greater clearances between the tens pins and the units rails, making the device less likely to jam.

In a further embodiment, the counting arrangement comprises an end-of-life lockout mechanism for preventing further rotation of the tens wheel, once the tens wheel has reached its end-of-life position. This optional end-of-life lockout mechanism comprises a tens wheel lockout pin or lockout tab that interacts with a feature on a stationary element of the counting device. The stationary element of the counting device is preferably a lockout wall or lockout cam on the housing component. The said lockout cam and the corresponding lockout pin on the opposite side of the tens wheel from the pins wreath together prevent rotation of the tens wheel in at least one intermediate configuration before the end-of-life lockout configuration is engaged.

According to another embodiment, an end-of-life lockout mechanism is provided for preventing the units wheel indexing cam face from engaging with the tens wheel pins wreath, once the tens wheel has reached its end-of-life position. Such a sub-mechanism preferably comprises one or more partial pins within the pins wreath. Such a partial pin may have a reduced height, for example a half height relative to the remaining pins of the pins wreath. Alternatively or in addition, the cross-section of the said partial pin may be less than the cross-section of a full pin. The reduced form of the partial pin, optionally combined with a deformation of the partial pin during an attempted extra index, allows the partial pin to avoid or skip past the indexing boss of the units wheel. In the case where deformation occurs to effect the missed indexing, an appropriate material should be used for the tens wheel to ensure that the partial pin deforms without cracking or separating from the remainder of the tens wheel.

### Detailed description

For a better understanding of the invention and to show how a preferred embodiment of the same may be carried into effect, reference will now be made, by way of example, of the accompanying drawings, in which:
- Fig. 1: shows an exploded view of the counting arrangement from a first point of view,
- Fig. 2: shows an exploded view of the counting arrangement from a second point of view,
- Fig. 3a: shows a front side view of the units wheel,
- Fig. 3b: shows a rear view of the units wheel,
- Fig. 4a: shows a front side view of the tens wheel,
- Fig. 4b: shows a rear view of the tens wheel,
- Figs. 5a-5f: show a front side view of the counting arrangement in the mounted state without front cover during the actuation steps, and
- Figs. Sa'-Sf': show a rear view of the counting arrangement in the mounting state without a gearbox chassis (back cover) during the actuation steps.

It is to be understood that the various features that are described in the following and/or illustrated in the drawings are preferred but not essential. Combinations of features described and/or illustrated are not considered to be the only possible combinations. Unless stated to the contrary, individual features may be omitted, variated or combined in different combinations, where practical.

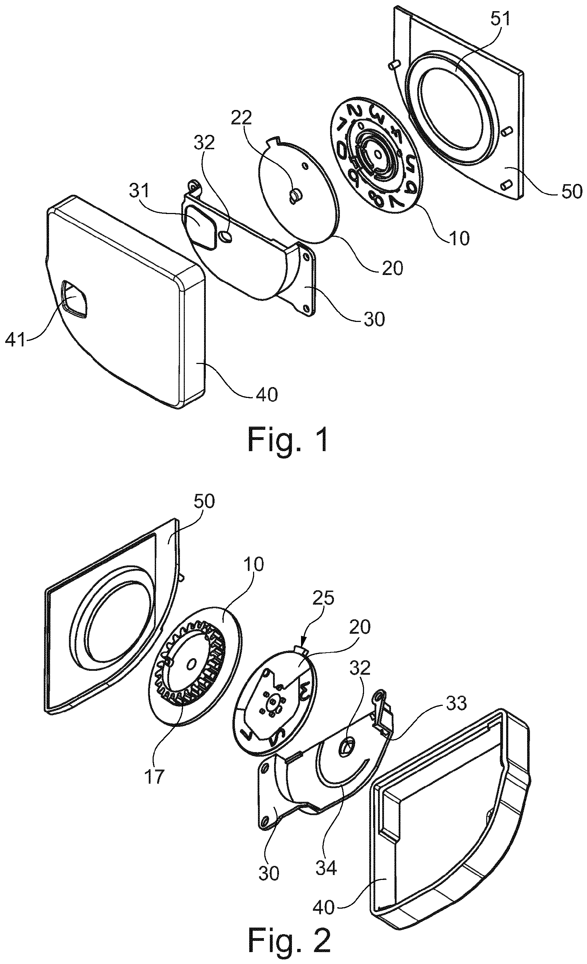

Fig. 1 illustrates a counting arrangement in an exploded view which consists of five single parts, namely a units wheel 10, tens wheel 20, housing component 30, front cover 40 and gearbox chassis 50. The gearbox chassis 50 comprises a guiding ring 51 to hold the adjacent units wheel 10. The bridge-shaped housing component 30 is attached to the gearbox chassis 50 and accommodates the partly overlapped arrangement of the units wheel 10 and the tens wheel 20 which in combination provides a two-digit display.

To recognize the display from outside, the housing component 30 is provided with a housing window 31. The housing component 30 further comprises a slot 32 for guiding an axis 22 of the tens wheel 20. The front cover 40 is also attached to the gearbox chassis 50 for enclosing the previously described inner components. The front cover 40 is provided with a housing window 41 which corresponds to the housing window 31 of the housing component 30.

Fig. 2 shows the rear sides of all the components described above. The housing component 30 further comprises a lockout wall 34 as a part of an end-of-life lockout mechanism which partly surrounds the central slot 32. A lockout edge 33 is also a part of the said end-of-life lockout mechanism. It corresponds to a lockout tab 25 formed on the outer circumferential edge of the tens wheel 20. On the rear side of the units wheel 10, a gear 17 is coaxially formed. The coaxially formed gear 17 of the units wheel 10 engages with drive means (not shown) of an inhaler device to link the dose count to the operation of the device.

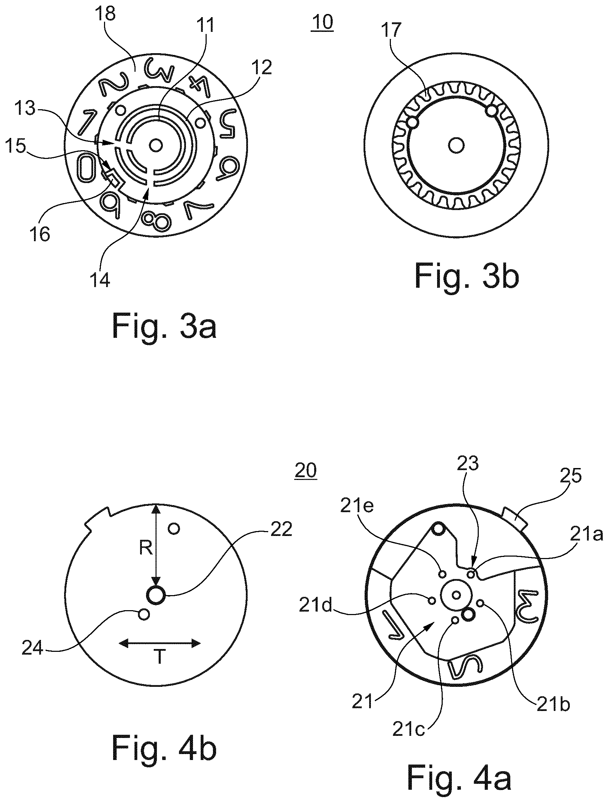

Figs. 3a and 3b show the units wheel 10 as a single component. The front side of the units wheel 10 comprises a recess for receiving the tens wheel pins wreath and axis, and this recess contains features of a rotational control mechanism according to the present invention. The units wheel comprises two circular rails 11 and 12 each with two radial rail gaps 13 and 14. The circular rails 11 and 12 enclose a units wheel tens-positioning cam 16. An indexing feature with an indexing cam face 15 is located at the outer edge of the recess XX. This feature is symmetrically arranged in radial direction together with both rail gaps 13 and 14. The cam 16 is surrounded by an annular outer edge area 18 on which the digits 0 to 9 are printed.

Figs. 4a and 4b show both sides of the tens wheel 20. On the rear side of the tens wheel 20, a pins wreath 21 is arranged which consists of five axial pins 21a to 21e. The axial pins 21a to 21e are equidistantly arranged in a circle to one another for engaging with the circular rail of the corresponding units wheel (not shown).

Furthermore, on the tens wheel 20, end-of-life lockout mechanism features are provided comprising a lockout tab 25 that interacts with a feature on the housing component (not shown) for avoiding a further rotation of the tens wheel 20 after dose counting is complete. Additionally, the pins wreath 21 comprises one partial pin 23 (21a) of a reduced height comparing to the other pins 21b to 21e, which is also an end-of-life lockout mechanism feature. On the opposite side of the tens wheel 20, the central axis 22 is formed.

Figs. 5a to 5f illustrate the functionality of the counting arrangement as described above. The sequence of the Figures shows the interaction of the units wheel 10 with the overlapping tens wheel (partly shown) in order to count back from 30 doses to 29 doses. For carrying out such a tens step, the units wheel 10 as well as the tens wheel 20 are moved by one step as shown.

The Fig. 5a shows the position of both wheels before indexing. The counter displays 30 doses. Fig. 5b shows a position in which the units wheel indexing cam face is in contact with the adjacent pin of the pins wreath. Fig. 5c shows a halfway-through-index position in which the units wheel indexing cam feature is in between adjacent pins of the tens wheel pins wreath. From Fig. 5b to Fig. 5e, two of the pins of the pins wreath that had previously locked the tens wheel against rotation pass through the radial rail gaps of the circular rails. As shown in Fig. 5e, the units wheel indexing cam continuing indexing. At points during indexing, the side faces of the radial rail gaps of the circular rails may assist indexing by acting on the pins that are passing through them. In the position as shown in Fig. Sf, the indexing to 29 doses is completed.

Additionally, Figs. 5a' to 5f' show a rear-side view of the indexing sequence as described above.

In an alternative embodiment of the invention as described above, a version of a counting arrangement is disclosed comprising a rotatably mounted units wheel arranged to rotate by one unit for every input that is to be counted; a tens wheel in mechanical communication with the units wheel, and in part overlapping the units wheel in order to provide an at least two-digit display; a rotation control mechanism that rotates the tens wheel by one increment during every tenth increment of the units wheel; wherein the units wheel locks the tens wheel in a configuration in which it cannot rotate during the intermediate nine inputs; wherein the rotational axis of the tens wheel remains substantially stationary while the tens wheel is rotated by one increment. As in the previous described embodiment the position of the tens wheel axis is substantially defined in the radial direction by a first, radially-constraining component, and substantially defined in the tangential direction by a separate second, tangentially-constraining component. The position of the tens wheel axis is previously defined in the radial direction by a circumferential units wheel tens-positioning cam. The position of the tens wheel axis is previously defined in the tangential direction by a slot in a housing component.

### Reference signs

- 10: units wheel
- 11: first circular rail
- 12: second circular rail
- 13: first radial gap
- 14: second radial gap
- 15: indexing cam face
- 16: cam
- 17: gear
- 18: edge area (with numerals)

- 20: tens wheel
- 21: pins wreath
- 22: axis
- 23: partial pin
- 24: lockout pin
- 25: lockout tab

- 30: housing component
- 31: window
- 32: slot
- 33: lockout edge
- 34: lockout wall

- 40: front cover
- 41: window

- 50: gear box chassi

## Claims

1. Counting arrangement for counting binary mechanical inputs comprising:
- a rotatably mounted units wheel (10) arranged to rotate by one unit for every input that is to be counted;
- a tens wheel (20) in mechanical communication with the units wheel (10), and in part overlapping the units wheel (10) in order to provide an at least two-digit display;
- a rotation control mechanism, comprising:
- at least one circular rail (11; 12) on the units wheel (10) with at least two substantially radial rail gaps (13, 14);
- a pins wreath (21) arranged on the tens wheel (20) consisting of several axial pins (21a - 21e) equidistantly arranged in a circle to one another for engaging with the circular rail (11; 12);
- a units wheel indexing cam face (15) that engages with the pins wreath (21) in order to effect driving of the tens wheel (20) by one increment during every tenth increment of the units wheel (10);
wherein the at least one circular rail (11, 12) on the units wheel (10) locks the tens wheel (20) in a configuration in which it cannot rotate during the intermediate nine inputs;
**characterised in that:**
- the position of the tens wheel axis (22) is defined in the radial direction (R) by a first, radially-constraining component, and defined in the tangential direction (T) by a separate second, tangentially-constraining component.

2. Counting arrangement according to claim 1, wherein the position of the tens wheel axis (22) is defined in the radial direction (R) by a circumferential units wheel tens-positioning cam (16).

3. Counting arrangement according to claims 1-2, wherein the position of the tens wheel axis (22) is defined in the tangential direction (T) by a slot (32) in a housing component (30).

4. Counting arrangement according to claim 3, wherein the units wheel indexing cam face (15) forms part of the units wheel circular locking rail (11; 12).

5. Counting arrangement according to any of the preceding claims, further comprising an end-of-life lockout mechanism for preventing further rotation of the tens wheel (20) once the tens wheel has reached its end-of-life position, comprising a tens wheel lockout pin (24) or lockout tab (25) that interacts with a feature on a stationary element of the counting arrangement.

6. Counting arrangement according to claim 5, wherein the stationary element of the counting arrangement is a lockout wall (33) and/or lockout cam-track (34) on the housing component (30).

7. Counting arrangement according to claim 6, wherein the lockout cam-track (34) and the lockout pin (24) together prevent rotation of the tens wheel (20) in at least one intermediate configuration before the end-of-life lockout configuration is engaged.

8. Counting arrangement according to any of the previous claims, further comprising an end-of-life lockout mechanism for preventing the units wheel indexing cam face (15) from engaging with the tens wheel pins wreath (21) once the tens wheel has reached its end-of-life position.

9. Counting arrangement according to claim 8, wherein the sub-mechanism for preventing the units wheel indexing cam face (15) from engaging with the tens wheel pins wreath (21) comprises one or more partial pins (23) within the pins wreath (21).

10. Counting arrangement according to claim 9, wherein the one or more partial pins (23) comprises at least one pin of a reduced height.

11. Counting arrangement according to claim 9 or 10, wherein the one or more partial pins (23) comprises at least one pin with a cross-section comprising a partial section of the cross-section of a full pin.

12. Medical dispenser device, especially dry powder inhaler, incorporating the counting arrangement as defined in any one of the preceding claims.
